# EUROPEAN PATENT APPLICATION

(11) **EP 2 992 876 A1**
(43) Date of publication of application: **09.03.2016**
(21) Application number: 15183868.7
(22) Date of filing: 04.09.2015
(51) Int. Cl.: A61K 9/20, A61K 31/4985, A61K 31/64

(54) **PHARMACEUTICAL COMBINATIONS OF SITAGLIPTIN**

(30) Priority: 05.09.2014 TR 201410448
(71) Applicant: Sanovel Ilac Sanayi ve Ticaret A.S., 34460 Istanbul (TR)
(72) Inventor: MUTLU, Onur, 34460 Istanbul (TR); TÜRKYILMAZ, Ali, 34460 Istanbul (TR); YELKEN, Gülay, 34460 Istanbul (TR); SAYDAM, Mehtap, 34460 Istanbul (TR)
(74) Representative: Sevinç, Erkan

(57) **Abstract**

A pharmaceutical combination comprising sitagliptin or a pharmaceutically acceptable salt thereof in combination with a sulfonylurea.

## Description

### Field of Invention

The present inventi3on relates to a pharmaceutical combination comprising sitagliptin or a pharmaceutically acceptable salt thereof in combination with a sulfonylurea and at least one pharmaceutically acceptable excipient.

### Background of Invention

Sitagliptin is indicated as an adjunct to diet and exercise to improve glycemic control in adults with type 2 diabetes mellitus. It is an oral antihyperglycemic of the dipeptidyl peptidase-4 (DPP-4) inhibitor class. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Sitagliptin works by binding to DPP-4 and preventing it from breaking down the GLP-1 and GIP. This increases the levels of these hormones in the body and so increases their effect on controlling blood sugar.

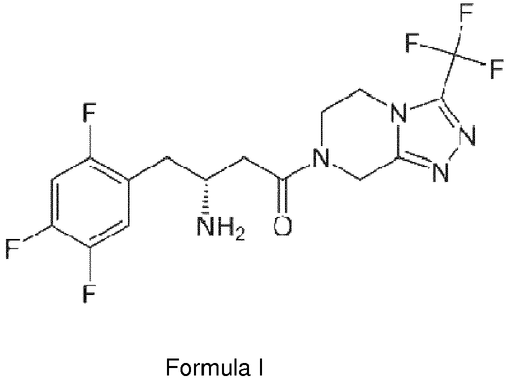

It is named as (3R)-3-amino-1-[3-(trifluoromethyl)-6,8-dihydro-5H-[1,2,4]triazolo[4,3-a]pyrazin-7-yl]-4-(2,4,5-trifluorophenyl)butan-1-one or (2R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine) and its chemical structure is shown in the Formula I.

Sitagliptin is licensed in the US and EU under the name of JANUVIA in the strength of 100 mg, 50 mg, and 25 mg. The recommended dose is 100 mg once daily.

DPP-4 inhibitors, especially sitagliptin have a novel mechanism of action, and many studies are available which shows their efficacy and safety in monotherapy or combination therapy. When the single sitagliptin alone does not provide adequate glycemic control, sulfonylureas are the most common co-administered drugs of it.

Sitagliptin increase plasma GLP-1 concentration and elevate cellular cAMP levels in pancreatic beta-cells leading to potentiate insulin secretion, whereas sulfonylurea drugs stimulate insulin secretion as a result of elevation of cytosolic Ca²⁺ concentration in the beta-cells. Therefore, they both help to reduce blood glucose levels in different pathways in type 2 diabetes patients and it is anticipated that a combination therapy of sitagliptin and sulfonylurea may synergistically stimulate insulin secretion and effectively ameliorate hyperglycemia in type 2 diabetes mellitus.

Sulfonylureas are divided into two categories: first generation drugs are comprising tolbutamide, chlorpropamide, tolazamide, acetohexamide and carbutamide; and second generation drugs are comprising glimepiride, gliclazide, glyburide (glibenclamide), glipizide, glibornuride, gliquidone, glisoxepide and glyclopyramide.

Glimepiride is a second generation sulfonylurea. It is used as an adjuvant to reduce blood sugar level in "diabetes mellitus type II" patients. As do all other sulfonylureas, it enhances the secretion of insulin from pancreatic beta cells and provides an insulin-like effect. The chemical name of glimepiride is 1-({p-(2-(3-ethyl-4-methyl-2-oxo-3-pyrroline-1-carboxamide)ethyl)phenyl}sulfonyl)-3-(trans-4-methylcyclohexyl)urea and the chemical structure is shown in Formula II.

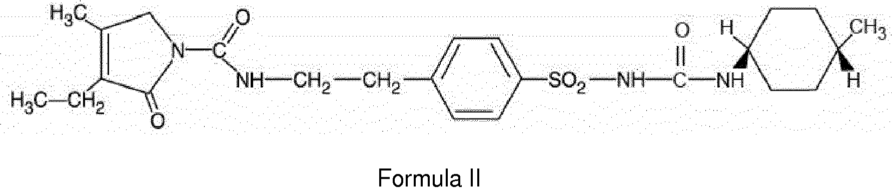

Glimepiride is marketed under the trademark AMARYL^{®} in 1, 2, and 4 mg dosage forms. It is used as an adjunct to diet and exercise to improve glycemic control in adults with type 2 diabetes mellitus.

In prior art, glimepiride molecule was first disclosed in the patent EP0031058.

Gliclazide N-(4-methylbenzenesulfonyl)-N'-(3-azabicyclo[3.3.0]-oct-3-yl)urea), one type of second generation sulfonylureas, is an active agent which has antidiabetic properties and its chemical structure is illustrated with Formula III.

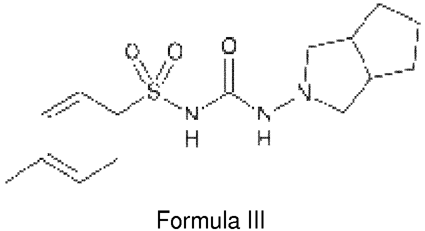

Gliclazide has been administered in the form of 80 mg conventional tablets. It is recommended to be administered one tablet twice a day.

The formulation of gliclazide was first disclosed in the patent document US 3501495 (SCIENCE UNION ET CIE. SOC.), with hypoglycemic properties, and is orally used in the treatment of diabetes mellitus.

In prior art, there are many pharmaceutical combination studies about sitagliptin. For instance, WO 2009/099734 discloses pharmaceutical compositions providing an extended release of metformin and an immediate release of sitagliptin. The tablet core is comprised of metformin and an extended release excipient (HPMC) (Hydroxypropyl methylcellulose). The tablet core is then coated with immediate release polymer comprising sitagliptin. However, in prior art, no study has been done in terms of sitagliptin and sulfonylurea combinations.

There is a need in the art for a pharmaceutical formulation or a dosage form that is to combine sitagliptin and sulfonylureas. However, some problems may be occurred while combining these two antidiabetic drugs such as, additional undesired side effects, physicochemical incompatibility and therapeutical incompatibility problems.

In this present invention, a pharmaceutical combination comprising sitagliptin or a pharmaceutically acceptable salt thereof in combination with a sulfonylurea has been developed to achieve a stable pharmaceutical combination therapy with a safe and effective release.

### Description of the invention

The main embodiment of this present invention is to provide a pharmaceutical combination comprising sitagliptin or a pharmaceutically acceptable salt thereof in combination with a sulfonylureas and at least one pharmaceutically acceptable excipient.

One embodiment of this present invention is to provide a pharmaceutical combination comprising sitagliptin and a sulfonylurea with an improved glycemic control in adults with diabetes mellitus.

According to one embodiment, the pharmaceutical combination is in the form of a tablet, bilayer tablet, multilayer tablet, mini tablet, capsule, gelatin capsule, oral granule, powder, sachet or a pellet.

In one embodiment, the sulfonylurea in the pharmaceutical combination of this present invention is selected from the group comprising glimepiride, gliclazide, glipizide, glibenclamide (glyburide), glibornuride, gliquidone, glisoxepide, glyclopyramide, tolbutamide, chlorpropamide, tolazamide, acetohexamide and carbutamide or pharmaceutically acceptable salts thereof. More preferably, the sulfonylurea is glimepiride or gliclazide.

In one embodiment, sulfonylureas used in the pharmaceutical combination of this present invention comprise first generation sulfonylureas and second generation sulfonylureas.

According to this embodiment, first generation sulfonylureas comprise tolbutamide, chlorpropamide, tolazamide, acetohexamide and carbutamide; second generation sulfonylureas comprise glimepiride, gliclazide, glipizide, glibenclamide (glyburide), glibornuride, gliquidone, glisoxepide and glyclopyramide.

While combining more than one molecule in one dosage form is increasing the patients' quality of life, many challenges also occur such as the physicochemical compatibility between the different active agents and/or between the active agents and the excipients used; and the therapeutical compatibility between the two active agents regarding their pharmacokinetic and/or pharmaceutical properties in order that the posology of the combined formulation allows to obtain safe and efficient plasma levels of both pharmacological agents.

It is well known that drugs even used in the same therapeutic area cannot always be combined in a dosage form. The scientific literature is full of examples wherein compounds of different classes, which are used to treat the same indications, cannot be combined into safe and efficacious dosage forms thereby resulting in incompatible drug combinations. The reasons for this unexpected lack of compatibility are varied; however, it is often found that the incompatible drug combinations result in increased side effects, unwanted drug interactions or additional side effects. More specifically, when sitagliptin is used in combination with a sulfonylurea, they may cause hypoglycemia as an additional side effect. Therefore, a lower dose of sulfonylurea is required to reduce the risk of hypoglycemia.

According to this embodiment, sitagliptin or a pharmaceutically acceptable salt thereof is present in an amount of between 1.5 - 90%, preferably 15 - 50% and more preferably 25 - 35% by weight of total formulation.

According to this embodiment, glimepiride or a pharmaceutically acceptable salt thereof is present in an amount of between 0.01 - 30%, preferably 0.01 - 20% and more preferably 0.01 - 16% by weight of total formulation.

According to this embodiment, gliclazide or a pharmaceutically acceptable salt thereof is present in an amount of between 0.01 - 70%, preferably 10 - 60% and more preferably 15 - 50% by weight of total formulation.

One embodiment of the present invention is to obtain a pharmaceutical combination comprising sitagliptin and a sulfonylurea which provides the desired release profile.

Another embodiment of the present invention is to obtain a stable pharmaceutical combination comprising sitagliptin and a sulfonylurea in one dosage form.

According to this embodiment, pharmaceutical combination of this present invention comprise at least one pharmaceutical excipient selected from the group comprising disintegrants, binders, diluents, glidants, lubricants or mixtures thereof.

Suitable disintegrants are selected from the group comprising polyvinylpyrrolidone (povidone), cross-linked polyvinylpyrrolidone (crospovidon or copovidon), croscarmellose sodium, low-substitue HPC (hydroxylpropyl cellulose), sodium starch glycolate, microcristalline cellulose, starch, alginic acid and alginates, ion-exchange resins, magnesium aluminum silica, sodium dodecyl sulphate, sodium carboxy methyl cellulose, carboxy methyl cellulose calcium, docusate sodium, guar gum, polyacrylin potasium, poloxomer, sodium alginate, sodium glysin carbonate, sodium lauryl sulphate or mixtures thereof.

Suitable binders are selected from the group comprising cross-linked polyvinylpyrrolidone, polyvinylpyrrolidone (povidone), polymethacrylate, glyceryl behenate, hydroxypropyl methyl cellulose (HPMC), hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose and other cellulose derivatives, polymetacrylates, polyethylene oxide, polyvinyl alcohol, polyvinyl acetate and their copolymers, gelatin, starch, xanthan gum, guar gum, alginate, pullulan, carrageen, kollagen, agar, pectin, hyaluronic acid, carbomer, cellulose acetate phthalate, hydroxypropyl starch, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose and mixtures thereof.

Suitable diluents are selected from the group comprising microcrystalline cellulose (MCC), mannitol, lactose, spray-dried lactose, sorbitol, sucrose, trehalose, isomalt, starch, calcium phosphate anhydrate, calcium phosphate dihydrate, calcium phosphate trihydrate, dibasic calcium phosphate, tribasic calcium phosphate, calcium carbonate, calcium sulfate, carboxymethyl cellulose calcium, powdered cellulose, cellulose acetate, pregelatinized starch, lactose monohydrate, sodium carbonate, sodium bicarbonate, maltodextrine, dextrose, isomalt, calcium carbonate, xylitol, corn starch or mixtures thereof.

Suitable glidants are selected from the group comprising colloidal silicon dioxide, talc, aluminum silicate, powdered cellulose, calcium phosphate tribasic, hydrophobic colloidal silica, magnesium oxide, magnesium trisilicate, magnesium silicate or mixtures thereof.

Suitable lubricants are selected from the group comprising magnesium stearate, calcium stearate, mineral oil, sodium stearyl fumarate, talc, polyethylene glycol, glyceryl monostearate, glyceryl palmitostearate, sodium lauryl sulphate, magnesium lauryl sulphate, fumaric acid, zinc stearate, stearic acid, hydrogenated natural oils, silica, paraffin or mixtures thereof.

Coating may also preferably be used for the combination. It can be selected from the group comprising polyvinyl alcohol-polyethylene glycol copolymers (Kollicoat IR), polyvinyl alcohol or copolymers or mixtures thereof (Opadry AMB), Ethylcellulose Dispersions (Surelease), Kerry-HPC, polyethylene glycol, polyvinylprolidone, polyvinylprolidone-vinyl acetate copolymer (PVP-VA) and all kinds of OpadryTM, as well as pigments, dyes, titanium dioxide, iron oxide, talc and polymethylmetacrylate copolymers (Eudragit).

In this embodiment, it is very essential to choose excipients used in the formulation. Both active agents should be stable with all excipients and each other. In general, DPP-4 inhibitors are not very stable compounds. Especially, in solid dosage forms, amine group containing DPP-4 inhibitors like sitagliptin may react with many excipients or impurities of excipients. To achieve the stability of both sitagliptin and sulfonylurea , selection of excipients and the ratio of them is very essential. The percentage of disintegrant is effective to achieve desired release profile with stable formulation. Especially polyvinylpyrrolidone is preferred as a disintegrant which is in the range of %0.25-10

In this present invention, to achieve desired pharmaceutical combination with a desired release profile, compatibility and stability, these formulations have been designed, comprising the following:
a. 1.5 - 90.0 % by weight of sitagliptin
b. 0.01 - 30% by weight of glimepiride
c. 0.1 - 25.0 % by weight of disintegrant
d. 0.1 - 25.0 % by weight of binder
e. 2.0 - 90.0 % by weight of diluent
f. 0.1 - 5.0 % by weight of glidant
g. 0.1 - 5.0 % by weight of lubricant
h. preferably, coating
and
a. 1.5 - 90.0 % by weight of sitagliptin
b. 0.01 - 70.0 % by weight of gliclazide
c. 0.1 - 25.0 % by weight of disintegrant
d. 0.1 - 25.0 % by weight of binder
e. 2.0 - 90.0 % by weight of diluent
f. 0.1 - 5.0 % by weight of glidant
g. 0.1 - 5.0 % by weight of lubricant
h. preferably, coating

### Example 1:

| **ingredients** | **Amount (%)** |
|---|---|
| sitagliptin | 1.5 - 90.0 |
| glimepiride | 0.01 - 16.0 |
| polyvinylpyrrolidone | 0.25 - 10 |
| microcrystalline cellulose | 10.0 - 85.0 |
| cross-linked polyvinylpyrrolidone | 0.2 - 20 |
| colloidal silicon dioxide | 0.1 - 5.0 |
| magnesium stearate | 0.1 - 5 |
| coating | 0.2 - 5.0 |

The production of the formulation is carried out as follows: microcrystalline cellulose is added to sitagliptin and glimepiride, and the resulting mixture is stirred. Polyvinylpyrrolidone and cross-linked polyvinylpyrrolidone are added to mixture and mixed together; thereby granulation is performed. The formed granules are dried, sieved; thereafter colloidal silicon dioxide and magnesium stearate are added therein and mixed together. The formed granules are compressed into tablets. At the final step, tablets are preferably coated.

### Example 2:

| **ingredients** | **Amount (%)** |
|---|---|
| sitagliptin | 1.5 - 90.0 |
| glimepiride | 0.01 - 16.0 |
| mannitol | 10.0 - 85.0 |
| microcrystalline cellulose | 2.0 - 90.0 |
| sodium starch glycolate | 0.2 - 20.0 |
| croscarmellose sodium | 0.2 - 20.0 |
| polyvinylpyrrolidone | 0.25 - 10.0 |
| colloidal silicon dioxide | 0.1 - 5.0 |
| magnesium stearate | 0.1 - 5.0 |
| coating | 0.2 - 5.0 |

The production of the formulation is carried out as follows: sitagliptin, glimepiride, mannitol, sodium starch glycolate and microcrystalline cellulose are mixed together. Then, polyvinylpyrrolidone dissolved in water/alcohol is sprayed to this mixture in a fluidized bed granulator. Coated granules are dried and sieved, croscarmellose sodium is added, the resulting mixture is mixed, magnesium stearate and colloidal silicon dioxide are added and mixed. At the final step, the mixture is compressed into tablets and tablets are preferably coated.

### Example 3:

| **ingredients** | **Amount (%)** |
|---|---|
| sitagliptin | 1.5 - 90.0 |
| glimepiride | 0.01 - 16.0 |
| microcrystalline cellulose | 10.0 - 85.0 |
| HPMC | 0.2 - 20.0 |
| polyvinylpyrrolidone | 0.25 - 10.0 |
| colloidal silicon dioxide | 0.1 - 5.0 |
| magnesium stearate | 0.1 - 5.0 |
| coating | 0.2 - 5.0 |

The production of the formulation is carried out as follows: Lactose, polyvinylpyrrolidone, HPMC and colloidal silicon dioxide are added to sitagliptin and glimepiride mixture. Magnesium stearate added to the resulting mixture and it is stirred and compressed into tablets. At the final step, tablets are preferably coated.

### Example 4:

| **ingredients** | **Amount (%)** |
|---|---|
| sitagliptin | 1.5 - 90.0 |
| gliclazide | 15.0 - 50.0 |
| mannitol | 5.0 - 90.0 |
| croscarmellose sodium | 6.0 - 25.0 |
| polyvinylpyrrolidone | 0.25 - 10.0 |
| colloidal silicon dioxide | 0.1 - 5.0 |
| magnesium stearate | 0.1 - 5.0 |
| coating | 0.2 - 5.0 |

The production of the formulation is carried out as follows: sitagliptin, gliclazide, croscarmellose sodium, and mannitol are sieved and mixed in a high-shear granulator. The resulting mixture is granulated with a polyvinylpyrrolidone solution. Wet granules formed are sieved, then dried and the dried granules are sieved again. First colloidal silicon dioxide and then magnesium stearate are added to granules. The resulting mixture is mixed. The obtained granules are filled into capsules or compressed into tablets. At the final step, tablets are preferably coated.

### Example 5:

| **ingredients** | **Amount (%)** |
|---|---|
| sitagliptin | 1.5 - 90.0 |
| gliclazide | 15.0 - 50.0 |
| lactose | 5.0 - 90.0 |
| HPMC | 0.2 - 20.0 |
| polyvinylpyrrolidone | 0.25 - 10.0 |
| colloidal silicon dioxide | 0.1 - 5.0 |
| magnesium stearate | 0.1 - 5.0 |
| coating | 0.2 - 5.0 |

The production of the formulation is carried out as follows: Sitagliptin, gliclazide, lactose and HPMC are sieved, and then mixed in a high-shear granulator. This mixture is granulated with a polyvinylpyrrolidone solution. Wet granules formed are sieved, then dried and the dried granules are sieved again. First colloidal silicon dioxide and then magnesium stearate are added to the granules and the resulting mixture is mixed. The granules and pellets obtained are filled into capsules to complete the process.

## Claims

1. A pharmaceutical combination comprising sitagliptin or a pharmaceutically acceptable salt thereof in combination with a sulfonylurea and at least one pharmaceutically acceptable excipient.

2. The pharmaceutical combination according to claim 1, wherein the sulfonylurea is selected from the group comprising glimepiride, gliclazide, glipizide, glyburide, glibornuride, gliquidone, glisoxepide, glyclopyramide, tolbutamide, chlorpropamide, tolazamide, acetohexamide and carbutamide, preferably glimepiride and gliclazide.

3. The pharmaceutical combination according to claim 1, wherein sitagliptin or a pharmaceutically acceptable salt thereof is present in an amount of between 1.5 - 90%, preferably 15 - 50% and more preferably 25 - 35% by weight of total formulation.

4. The pharmaceutical combination according to claim 2, wherein glimepiride or a pharmaceutically acceptable salt thereof is present in an amount of between 0.01 - 30%, preferably 0.01 - 20% and more preferably 0.01 - 16% by weight of total formulation.

5. The pharmaceutical combination according to claim 2, wherein gliclazide or a pharmaceutically acceptable salt thereof is present in an amount of between 0.01 - 70%, preferably 10 - 60% and more preferably 15 - 50% by weight of total formulation.

6. The pharmaceutical combination according to claim 1 is in the form of a tablet, bilayer tablet, multilayer tablet, mini tablet, capsule, gelatin capsule, oral granule, powder, sachet or a pellet.

7. The pharmaceutical combination according to claim 6, wherein at least one pharmaceutically acceptable excipient is selected from the group comprising disintegrants, binders, diluents, glidants, lubricants or mixtures thereof.

8. The pharmaceutical combination according to claim 7, wherein the disintegrant is present in an amount of between 0.25 - 10% by weight of total formulation.

9. The pharmaceutical combination according to claim 8, wherein the disintegrant is selected from the group comprising cross-linked polyvinylpyrrolidone, croscarmellose sodium, hydroxylpropyl methyl cellulose, low-substitue hydroxylpropyl cellulose, sodium starch glycolate, microcristalline cellulose, starch, alginic acid and alginates, ion-exchange resins, magnesium aluminum silica, sodium dodecyl sulphate, sodium carboxy methyl cellulose, carboxy methyl cellulose calcium, docusate sodium, guar gam, polyacrylin potasium, poloxomer, sodium alginate, sodium glysin carbonate, sodium lauryl sulphate or mixtures thereof.

10. The pharmaceutical combination according to claim 9, wherein disintegrant is polyvinylpyrrolidone.

11. The pharmaceutical combination according to any preceding claims comprising;
a. 1.5 - 90.0 % by weight of sitagliptin
b. 0.01 - 30% by weight of glimepiride
c. 0.1 - 25.0 % by weight of disintegrant
d. 0.1 - 25.0 % by weight of binder
e. 2.0 - 90.0 % by weight of diluent
f. 0.1 - 5.0 % by weight of glidant
g. 0.1 - 5.0 % by weight of lubricant
h. preferably, coating

12. The pharmaceutical combination according to any preceding claims comprising;
a. 1.5 - 90.0 % by weight of sitagliptin
b. 0.01 - 70.0 % by weight of gliclazide
c. 0.1 - 25.0 % by weight of disintegrant
d. 0.1 - 25.0 % by weight of binder
e. 2.0 - 90.0 % by weight of diluent
f. 0.1 - 5.0 % by weight of glidant
g. 0.1 - 5.0 % by weight of lubricant
h. preferably, coating
